# EUROPEAN PATENT APPLICATION

(11) **EP 4 600 742 A2**
(43) Date of publication of application: **13.08.2025**
(21) Application number: 25184062.5
(22) Date of filing: 29.05.2019
(51) Int. Cl.: G03F 7/039

(54) **INCREASING EFFICIENCY OF PHOTOCHEMICAL REACTIONS ON SUBSTRATES**

(30) Priority: 29.05.2018 US 201862677185 P
(62) Divisional of application: 19810959.7
(71) Applicant: Centrillion Technologies, Inc., Palo Alto CA 94303 (US)
(72) Inventor: MCGALL, Glenn, Palo Alto, 94303 (US); CRANDALL, Darren, Palo Alto, 94303 (US); LI, Bolan, Palo Alto, 94303 (US); DENTINGER, Paul, Palo Alto, 94303 (US)
(74) Representative: Hepworth Browne

(57) **Abstract**

Disclosed herein is a substrate which includes a functional group protected with a photolabile group covalently attached to the substrate and a film of solvent thereof covering the substrate, where the thickness of the film is less than about 100 µm. Also disclosed herein are methods of preparing such substrates. Further disclosed are methods of synthesizing polymers, methods of synthesizing arrays of polymers and methods of removing photolabile protecting groups. These methods all employ covering the substrate with a thin film of solvent where the thickness of the film is less than 100 µm.

## Description

### CROSS REFERENCE TO RELATED APPLICATIONS

This application claims priority under 35 U.S.C. § 119 (e) from United States Provisional Application Serial No. 62/677,185 filed May 29, 2018, which is hereby incorporated by reference for all purposes in its entirety.

### FIELD

Disclosed herein is a substrate which includes a functional group protected with a photolabile group covalently attached to the substrate and a film of solvent thereof covering the substrate, where the thickness of the film is less than about 100 µm. Also disclosed herein are methods of preparing such substrates. Further disclosed are methods of synthesizing polymers, methods of synthesizing arrays of polymers and methods of removing photolabile protecting groups. These methods all employ covering the substrate with a thin film of solvent where the thickness of the film is less than 100 µm.

### BACKGROUND

High-density DNA microarrays have seen extensive use in a range of genomics applications, including the detection and analysis of genetic mutations and polymorphisms (Matsuzaki H., et al., 2004, Nature Methods, 1, 109-111; and Gunderson K.L., et al., 2005 Nature Genetics, 37(5), 549-554); cytogenetics Hudgins, M. M. et al., 2010, Genetics in Medicine, 12(11), 742-745; Collas, P., 2010, Molecular Biotechnology, 45(1), 87-100; Maruyama K., et al., Methods Mol Biol. 2014, 1062, 381-91, doi: 10.1007/978-1-62703-580-4-20; Su A.I., et al., 2002, Proceeding of the National Academy of Sciences, 99(7), 4465-4470; and Kosuri S., Church, G. M., 2014, Nature Methods 2014, 11(5), 499).

DNA oligonucleotide microarrays have been fabricated on a commercial scale by direct, in-situ synthesis on chip substrates using photolithography (Pease A.C., et al., 1994, Proceeding of the National Academy of Sciences, 5022-5026; McGall G.H., et al. J. Am. Chem. Soc. (1997) 119, 5081, Singh-Gasson S., et al., Nature Biotechnology 1999, 17, 974-978; and Pawloski A.R. et al., 2007, Journal of Vacuum Science and Technology B, 25(6), 2537-2456); inkjet printing (Hughes T.R. et al., 2001, Nature Biotechnology 19: 342-347, Lausted C, et al., 2004, Genome Biology 5: R58); and (LeProust et al., 2010, Nucleic Acids Research 38: 2522-2540); electrochemistry, Liu R.H., et al., 2006, Expert Reviews of Molecular Diagnostics 6: 253-261; and other methods; Gunderson, K.L. et al., Genome Research 2004. 14, 870-877).

Fabricating DNA microarrays using in situ synthesis and photolithography provides a flexible, economical means of manufacturing arrays with densities greater than 10⁷ distinct sequences per cm². This method employs photoremovable protecting groups such as, for example, MeNPOC, NNBOC, DMBOC, PYMOC, NPPOC, DEACMOC, SPh-NPPOC, etc., some of which are illustrated below:

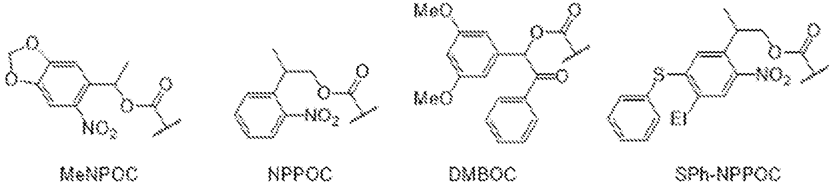

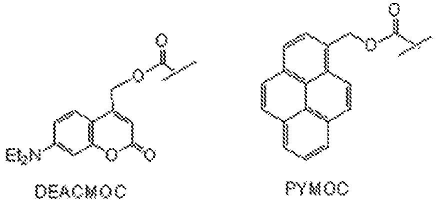

The above groups undergo photolytic reactions to effect their release. In many cases, these reactions require co-reactants or catalysts to promote the photocleavage reaction. For example, photoreleasable NPPOC groups require a base to catalyze a photo-elimination reaction. Arylmethyl groups such as PyMOC and DEACMOC, a polar, nucleophilic solvent is required to promote a photo-solvolysis reaction (McGall et al., U.S. Patent Application No. US20110028350). Photolithographic synthesis of DNA arrays using a solution of at least one photosensitizer to catalyze deprotection reactions has also been described (McGall et al., U.S. Patent No. 7,144,700).

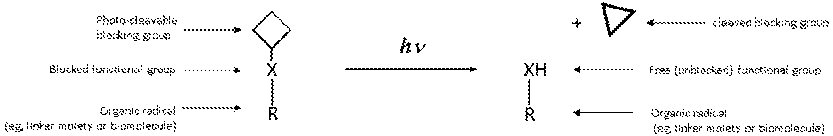

In the above situations, photolithographic exposure requires a film including a co-reactant or catalyst on the substrate which encompasses the attached photoremovable protecting groups. In the absence of solvents, co-reactants or catalysts, photolytic deprotection on surfaces may be relatively inefficient and subject to side-reactions that reduce the yield of the desired product (McGall G.H., et al. J. Am. Chem. Soc. (1997) 119, 5081).

High-throughput array manufacturing, for the above reasons, is limited to the options for suitable photolithographic exposure tools ("aligners") to systems which use either projection or proximity lithography. In these systems, the image-defining photomask is maintained some distance away from the surface being exposed. This is necessary since direct contact between the mask and the photolysis-promoting liquid or film would introduce undesirable optical image distortion which interferes with the alignment of the mask and wafer; degrades the image reaching the wafer surface; and contaminates the mask with coating material.

Modern projection or proximity lithography systems have co-evolved with the development of polymeric photoresists designed to serve for the microelectronics and semiconductor microchip industries. Photoresist processes have a non-linear or "threshold" response to light intensity, and this mitigates the normal resolution-degradation due to the low optical contrast, and "flare" that are characteristic of these optical systems. Unlike modern photoresists, photolabile protecting group removal is linearly dependent on light intensity. Sharp, image-wise photolysis is therefore intolerant of stray light from diffraction and flare.

Photolytic ortho-nitrobenzyl protecting groups undergo an intramolecular photo-redox reaction which does not require co-reactants or catalysts as shown below. Accordingly, in principle, the photolysis of nitrobenzyl groups can be performed on substrate surfaces in a dry state, which would enable the use of contact photolithography to transfer very high-contrast, high-resolution images to the substrate surface. With contact lithography, the photomask is brought in direct, "hard" contact with the surface of the wafer.

Typically, a vacuum is introduced between the mask and the substrate in order to maintain a minimum distance between wafer and mask, which prevents diffraction and flare from degrading the quality of the light image reaching the substrate. This is a major advantage for manufacturing, as the image-wise removal of nitrobenzyl and other photo-removable protecting groups (being linearly dependent on light intensity) is intolerant of stray light from diffraction and "flare", which are typical of projection or proximity systems (Introduction to Microlithography, 2nd ed., edited by L. F. Thompson, C. G. Willson, and M. J. Bowden. American Chemical Society, Washington, DC, 1994).

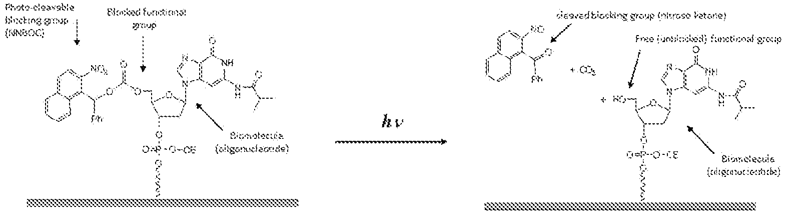

Unexpectedly, we have found that the efficiency of photolysis of ortho-nitrobenzyl protecting groups , for example, is, in fact, significantly reduced when carried out on a surface in a fully dry state. This effect appears to be due to the buildup of an immobile layer of photolysis products on the surface which slows or inhibits the productive absorption of light energy by those molecules which are not yet photolyzed.

However, in the presence of most organic solvents, the yield of full-length product is high (>95%) which suggests that solvent promotes diffusion of photolysis products away from the substrate surface during the exposure step, thus improving photolytic efficiency. Under dry conditions, the efficiency of removing photolabile groups is significantly compromised, thereby reducing the yield of full-length product.

However, when performing front-side exposures on contact aligners for photolithographic synthesis, deployment of a bulk solvent layer between the mask and the substrate which array synthesis is impractical. Some improvement in synthesis yield can be achieved by using dry exposure on an aligner by substantially increasing the exposure dose (i.e., by exposing longer), and frequently interrupting exposure to remove and wash the substrate free of accumulated photolysis product. However, the additional manipulation and UV exposure results in an unacceptable decrease in process throughput as well as image quality and synthesized polymer purity.

Thus, there is an unmet need to develop materials and processes to replace bulk solvents by application of very thin, stable liquid films, containing, if necessary, the required catalysts or co-reactants for efficient release of photochemical protecting groups, in order to fully exploit the tools and protocols available for high-resolution photolithographic array fabrication, including for example, contact photolithography. Ideally, such materials and processes will greatly increase the efficiency of photochemical reactions on a substrate and thus the stepwise yield of polymer synthesis on substrates where a photochemical reaction is an essential step.

### SUMMARY

The present invention satisfies these and other needs by providing materials and processes which increase the efficiency of photochemical reactions on a substrate. The materials and processes described herein also improve the stepwise yield of polymer synthesis on substrates where a photochemical reaction on a solid phase is a necessary step.

In one aspect, a substrate including a functional group protected with a photolabile group covalently attached to the substrate is provided. A film of solvent covers the substrate, where the thickness of the film is less than about 100 µm.

In another aspect, a method of making a substrate comprising a functional group protected with a photolabile group covalently attached to the substrate coated with a film of solvent is provided. The method includes the step of comprising spin coating the substrate with the film, where the thickness of the film is less than about100 µm.

In still another aspect, a method of synthesizing a polymer on a substrate is provided. The method includes the steps of providing a substrate comprising functional groups protected by a photolabile protecting group, coating the substrate with a film of solvent wherein thee thickness of the film is less than about 100 µm, irradiating the photolabile group, removing the film; coupling another monomer having a functional group protected with a photolabile group to the functional group and repeating steps b, c, d and e for one or more repetitions to synthesize the polymer.

In still another aspect, a method of synthesizing an array of polymers is provided. The method includes the steps of providing a substrate comprising functional groups protected by a photolabile protecting group, coating the substrate with a film of solvent wherein the thickness of the film is less than about 100 µm irradiating a selected region of the substrate to remove the photolabile group, removing the film, coupling monomers having a functional group protected with a photolabile group to the functional group and repeating steps b, d and e for one or more repetitions with a different selected region of the substrate to synthesis the array of polymers.

In still another aspect, a method of removing a photolabile group from a functional group of a monomer covalently attached to a substrate is provided The method includes the steps of coating the substrate with a thin film of solvent where the thickness of the film is than about 100 µm and irradiating the photolabile group.

### BRIEF DESCRIPTION OF THE DRAWINGS

Fig. 1A illustrates film transfer from the substrate to the mask during contact lithography when the substrate lacks offsets.
Fig. 1B illustrates the lack of film transfer from the substrate to the mask during contact lithography when the substrate includes offsets.
Fig. 2 illustrates a flow chart which describes the synthesis and HLPC analysis of the oligo Tₙ trimers made herein.
Fig. 3 illustrates the structure of reagents used in the processes described in Fig. 2
Fig. 4A illustrates HPLC analysis of T₃ synthesis when propylene carbonate is used as a bulk solvent.
Fig. 4B illustrates HPLC analysis of T₃ synthesis when 10% propylene carbonate in PGMEA is applied as a thin film of about 250 nm thickness.
Fig. 5 illustrates HPLC analysis of T₅ synthesis when no film is used.
Fig. 6 illustrates HPLC analysis of T₅ synthesis with propylene carbonate and acetonitrile film.
Fig. 7 illustrates HPLC analysis of T₅ synthesis with diethyleneglycol ethylhexyl ether and propylene glycol methyl ether acetate film
Fig. 8 illustrates HPLC analysis of T₁₀ synthesis with no film.
Fig. 9 illustrates HPLC analysis of T₁₀ synthesis with diethyleneglycol ethylhexyl ether and propylene glycol methyl ether acetate film.
Fig. 10 illustrates HPLC analysis of T₁₀ synthesis with 3-(2-(2-ethylhexyloxy)ethoxypropanenitrile and propylene glycol methyl ether acetate film.
Fig. 11 illustrates HPLC analysis of T₁₀ synthesis with surfynol 440 and propylene glycol methyl ether acetate film.
Fig. 12 illustrates HPLC analysis of T₁₀ synthesis with surfynol 440 and 3-methylglutaronitrile and propylene glycol methyl ether acetate film.
Fig. 13 illustrates HPLC analysis of T₁₀ synthesis with surfynol 440, 4-methoxymethyl-1,3-dioxolan-2-one (r-(+)) and propylene glycol methyl ether acetate film.
Fig. 14 illustrates HPLC analysis of T₁₀ synthesis with surfynol 440, diethyl phthalate and propylene glycol methyl ether acetate film.
Fig. 15 illustrates HPLC analysis of T₁₀ synthesis with surfynol 440, 1.0% bis(2-cyanoethyl ether) and propylene glycol methyl ether acetate film

### DETAILED DESCRIPTION

### DEFINITIONS

The singular form "a," "an," and "the" include plural references unless the context clearly dictates otherwise. The term "an agent," for example, includes a plurality of agents, including mixtures thereof.

Descriptions in range formats are provided merely for brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6, etc., as well as individual numbers within that range, for example, 1, 2, 3, 4, 5, and 6. This applies regardless of the breadth of the range.

"Array" as used herein is a preselected collection of different polymer sequences or probes which are associated with a surface of a substrate. An array may include polymers of a given length having all possible monomer sequences made up of a specific basis set of monomers, or a specific subset of such an array. For example, an array of all possible oligonucleotides of length 8 includes 65,536 different sequences. However, as noted above, an oligonucleotide array also may include only a subset of the complete set of probes. Similarly, a given array may exist on more than one separate substrate, e.g., where the number of sequences necessitates a larger surface area in order to include all of the desired polymer sequences.

"Chemically-removable protecting group" as used herein as is a group that blocks a reactive site in a molecule while a chemical reaction is carried out at another reactive site, and which is removable by exposure to a chemical agent, that is by means other than exposure to radiation. For example, one type of chemically-removable protecting group is removable by exposure to a base (i.e., "base-removable protecting groups"). Examples of specific base-removable protecting groups include, but are not limited to, fluorenylmethyloxycarbonyl (FMOC), 2-cyanoethyl (CE), N-trifluoroacetylaminoethyl (TF), 2-(4-nitrophenyl)ethyl (NPE), and 2-(4-nitrophenyl)ethyloxycarbonyl (NPEOC). Exocyclic amine groups on nucleotides; in particular on phosphoramidites, are preferably protected by dimethylformamidine on the adenosine and guanosine bases, and isobutyryl on the cytidine bases, both of which are base labile protecting groups. Another type of chemically removable protecting groups are removable by exposure to a nucleophile (i.e., "nucleophile-removable protecting groups"). Specific examples of nucleophile-removable protecting groups including but are not limited to levulinyl (Lev) and aryloxycarbonyl (AOC). Other chemically-removable protecting groups are removable by exposure to an acid (i.e., "acid-removable protecting groups"). Specific acid-removable protecting groups include but are not limited to triphenylmethyl (Tr or trityl), 4-methoxytriphenylmethyl (MMT or monomethoxytrityl), 4,4'-dimethoxytriphenylmethyl (DMT or dimethoxytrityl), tert-butoxycarbonyl (tBOC), α,α-dimethyl-3,5-dimethyoxybenzyloxycarbonyl (DDz), 2-(trimethylsilyl)ethyl (TMSE), benzyloxycarbonyl (CBZ), dimethoxytrityl (DMT), and 2-(trimethylsilyl)ethyloxycarbonyl (TMSEOC). Another type of chemically-removable protecting group is removable by exposure to a reductant (i.e., "reductant-removable protecting group"). Specific examples of reductant-removable protecting groups include 2-anthraquinonylmethyloxycarbonyl (AQMOC) and 2,2,2-trichloroethyloxycarbonyl (TROC). Additional examples of chemically-removable protecting groups include allyl (All) and allyloxycarbonyl (AIIOC) protecting groups.

"Film" as used herein refers to a layer or coating having one or more constituents, applied in a generally uniform manner over the entire surface of a substrate, for example, by spin coating. A film may be a solvent, mixtures of solvents, solutions or suspensions. For example, a film can include a photoacid generator and optionally a base and a sensitizer or a film may be a mixture of two or more different solvents.

"Feature" as used herein is as a selected region on a surface of a substrate in which a given polymer sequence is contained. Thus, where an array contains, e.g., 100,000 different positionally distinct polymer sequences on a single substrate, there will be 100,000 features.

"Functional group as used herein is a reactive chemical moiety present on a given monomer, polymer or substrate surface. Examples of functional groups include, e.g., the 3' and 5' hydroxyl groups of nucleotides and nucleosides, as well as the reactive groups on the nucleobases of the nucleic acid monomers, e.g., the exocyclic amine group of guanosine, as well as amino and carboxyl groups on amino acid monomers.

"Linker" as used herein can be any molecule linked to a substrate which distance the site of polymer synthesis from the substrate of a surface. The linker should be about 4 to about 40 atoms long and may be, for example, aryl acetylene, ethylene glycol oligomers containing 2-10 monomer units, diamines, diacids, amino acids, among others, and combinations thereof. Alternatively, the linkers may be the same molecule type as that being synthesized (i.e., nascent polymers), such as oligonucleotides or oligopeptides. The linker used herein may be provided with a functional group to which is bound a protective group.

"Monomer" as used herein is a member of the set of smaller molecules which can be joined together to form a larger molecule or polymer. The set of monomers includes but is not restricted to, for example, the set of common L-amino acids, the set of D-amino acids, the set of natural or synthetic amino acids, the set of nucleotides (both ribonucleotides and deoxyribonucleotides, natural and unnatural) and the set of pentoses and hexoses. Accordingly, monomer refers to any member of a basis set for synthesis of a larger molecule. A selected set of monomers forms a basis set of monomers. For example, the basis set of nucleotides includes A, T (or U), G and C. In another example, dimers of the 20 naturally occurring L-amino acids form a basis set of 400 monomers for synthesis of polypeptides. Different basis sets of monomers may be used in any of the successive steps in the synthesis of a polymer. Furthermore, each of the sets may include protected members which are modified after synthesis.

"Photoacid generator" as used herein is a compound or substance which produces acid (H⁺ or H₃O⁺) upon exposure to light having a predetermined wavelength.

"Photolabile group" as used herein is a group that block a reactive site on a molecule while a chemical reaction is carried out at another reactive site, and which is removable by exposure to radiation such as light radiation (see, e.g., Pelliccioli & Wirz, Photochem. Photobiol. Sci. 2002, 1:441-458; Bochet, J. Chem. Soc., Perkin Trans. 12002, 125-142). Specific examples of photolabile protecting groups for amines, thiols and hydroxyl groups include, but are not limited to, dimethoxybenzoin, 2-nitroveratryloxycarbonyl (NVOC); α-methyl-2-nitroveratryloxycarbonyl (MeNVOC); 2-nitropiperonyloxycarbonyl (NPOC); α-methyl-2-nitropiperonyloxycarbonyl (MeNPOC); 2-nitronaphth-1-ylmethyloxycarbonyl (NNPOC); 2-nitronaphth-1-ylbenzyloxycarbonyl (NNBOC); 6,-methoxy, 2-nitronaphth-1-ylbenzyloxycarbonyl (6-methoxy NNBOC); α-methyl-2-nitronaphth-1-ylmethyloxycarbonyl; α-phenyl-2-nitronaphth-1-ylmethyloxycarbonyl; 2,6-dinitrobenzyloxycarbonyl (DNBOC), α-methyl-2,6-dinitrobenzyloxycarbonyl (MeDNBOC); α-phenyl-2-nitroveratryloxycarbonyl (MeNVOC); phenyl-2-nitropiperonyloxycarbonyl (MeNPOC); 2-(2-nitrophenyl)ethyloxycarbonyl (NPEOC), 2-methyl-2-(2-nitrophenyl)ethyloxycarbonyl (NPPOC); 1-pyrenylmethyloxycarbonyl (PYMOC), 9-anthracenylmethyloxycarbonyl (ANMOC); 7-methoxycoumarin-4-ylmethyloxycarbonyl (MCMOC); 6,7-dimethoxycoumarin-4-ylmethyloxycarbonyl (DMCMOC); 7-(N,N-diethylamino)coumarin-4-ylmethyloxycarbonyl (DEACMOC); 3' methoxybenzoinyloxycarbonyl (MBOC), 3',5'-dimethoxybenzoinyloxycarbonyl (DMBOC), 7-nitroindolinyloxycarbonyl (NIOC), 5-bromo-7-nitroindolinyloxycarbonyl (BNIOC), 5,7-dinitroindolinyloxycarbonyl (DNIOC), 2-anthraquinonylmethyloxycarbonyl (AQMOC), α,α-dimethyl-3,5-dimethoxybenzyloxycarbonyl. The non-carbonate, benzylic forms of any of the foregoing, e.g., nitroveratryl (NV), α-methyl nitroveratryl (MeNV), etc., can be used for the protection of carboxylic acids as well as for amines, thiols and hydroxyl groups. Additional photoprotecting groups include all isomers of NNBOC, which may optionally substituted with 1 or more methoxy groups. Additional useful protecting groups include the below structures and all regioisomers thereof which may be optionally substituted with one or more methoxy groups.

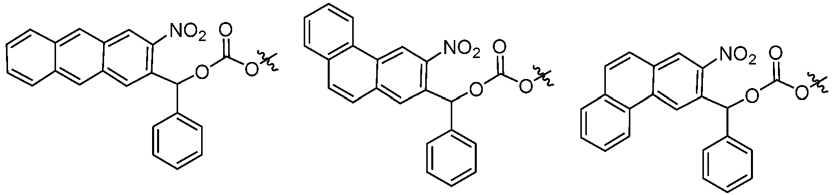

"Predefined region" as used herein is a localized area on a substrate which is, was, or is intended to be used for formation of a selected polymer and is otherwise referred to herein in the alternative as "reaction" region, a "selected" region, simply a "region" or a "feature." The predefined region may have any convenient shape, e.g., circular, rectangular, elliptical, wedge-shaped, etc. The arrays described herein have features on the order of 10-100 µm, i.e. 10x10 µm² to 100x10 µm² for approximately square features. The features may be between 1-10 µm or between 100-1000 nm. Within these regions, the polymer synthesized therein is preferably synthesized in a substantially pure form. However, in other embodiments a predefined regions may substantially overlap. In such embodiments, hybridization results may be resolved by software for example.

"Protecting group" as used herein is a group that blocks a reactive site on a molecule but can be removed on exposure to an activator or a deprotecting agent. Activators include, for example, electromagnetic radiation, ion beams, electric fields, magnetic fields, electron beams, x-ray, and the like. A deprotecting agent is a chemical or agent which causes a protective group to be cleaved from a protected group. Deprotecting agents include, for example, an acid, a base or a free radical. A deprotecting agent can be an activatable deprotecting agent. An activatable deprotecting agent is a chemical or agent which is relatively inert with respect to a protective group, i.e., the activatable deprotecting agent will not cause cleavage of the protective group in any significant amount absent activation. An activatable deprotecting agent may be activated in a variety of ways depending on its chemical and physical properties. Some activatable deprotecting agents may be activated by exposure to some form of activator, e.g. electromagnetic radiation. Some activatable deprotecting agent will be activatable at only certain wave lengths of electromagnetic radiation and not at others. For example, certain activatable deprotecting reagents will be activated with visible or UV light. In some cases, a deprotecting agent can be a vapor phase deprotection agents, which can be introduced at low pressure, atmospheric pressure, among others.

"Self-assembled monolayer" as used herein includes at least one type of linker. This linker has a backbone chain of carbon atoms, a head group at one end of the backbone for attachment to the surface of a substrate and a tail group at the other end to provide a support for polymer array synthesis. This linker is sometimes referred to as a functional linker or functional SAM linker in distinction from a non-functional linker, which can form a SAM but lacks a tail group to provide a site for further attachment. Array polymers can attach directly to the tail group or indirectly via an extension linker of the functional SAM linker. The tail group is preferably protected or inactivated or otherwise in precursor form during formation of the monolayer but deprotected or activated or otherwise rendered functional before array synthesis or attaching an extension or in situ synthesized linker.

"Sensitizer" as used herein is a compound which aids in the use of certain photoacid generators ("PAGs"). The sensitizer may extend the photosensitivity of the PAG, i.e. to shift the photo sensitivity to a longer wavelength of electromagnetic radiation. The sensitizer, also called a photosensitizer, may activate the PAG at, for example, at a longer wavelength of light if the concentration of the sensitizer is greater than that of the PAG, such as 1.1 times to 5 times greater, for example, 1.1 times to 3 times greater the concentration of PAG. Exemplary sensitizers suitable include isopropylthioxanthone (ITX) and 10H-phenoxazine (PhX).

"Substrate" as used herein is a material or group of materials having a rigid, semi-rigid surface or flexible surface suitable for attaching an array of polymers, particularly an array of nucleic acids. Suitable materials include polymers, plastics, resins, polysaccharides, silica or silica-based materials, carbon, metals, inorganic glasses, membranes. In some embodiments, the substrate is silicon, quartz or fused silica.. The surface can be the same or different material as the rest of the substrate. In some substrates, at least one surface of the substrate is flat, although in some substrates it may be desirable to physically separate synthesis regions for different compounds with, for example, wells, raised regions, pins, etched trenches, or the like. The substrate can take the form of beads, resins, gels, microspheres, or other geometric configurations. (See, e.g., U.S. Patent Nos. 5,744,305, 7,745,091, 7,745,092 and U.S. Patent Application Publication Nos. US20100290018, US20100227279, US20100227770, US20100297336, and US20100297448 for exemplary substrates and microspheres. In some embodiments, the substrate may include an offset. In these embodiments, the offset is typically a metal or metal oxide. The metal or metal oxide may be, for example, gold tungsten, tantalum, chromium or mixtures. In general the offset should be inert to all array fabrication conditions and reagents.

### THIN FILMS MATERIALS AND METHODS

Disclosed herein is a substrate which includes a functional group protected with a photolabile group covalently attached to the substrate and a film of solvent covering the substrate, where the thickness of the film is less than about 100 µm. Also disclosed herein are methods of preparing such substrates. Further disclosed are methods of synthesizing polymers, methods of synthesizing arrays of polymers and methods of removing photolabile protecting groups. These methods all employ covering the substrate with a film of solvent where the thickness of the film is less than about 100 µm.

The substrate may include a functional group covalently attached to a self-assembled monolayer on the substrate. The substrate also may include a functional group attached to a monomer, which is covalently attached to a self-assembled monolayer on the substrate.

The substrate may include a functional group covalently attached to a linker covalently attached to the substrate. The substrate may also include a functional group covalently attached to a monomer, which is covalently attached to a linker covalently attached to the substrate.

Disclosed herein is application of a thin, uniform film onto a substrate surface may provide the same improvement in yield and efficiency as deploying bulk solvent on the substrate surface. However, problems associated with use of bulk solvents, e.g., poor image resolution, low synthesis yields and possible contamination of the mask are avoided.

Film thickness is an important variable and may vary widely depending on the specific applications. For example, in various maskless lithographic methods, films of thickness of about less than 100 µm may be useful. When using contact aligners, films of less than about 100 nm may be desirable. The thickness of the film may be between about 100 µm and about 100 nm, between about 100 nm and about 50 nm, between about 100 nm and about 50 nm.

Acceptable films must have very low vapor pressure (i.e., high-boiling point) in order not to evaporate under ambient conditions but have sufficiently low viscosity to enable diffusion of photolysis products away from the substrate surface. The film should spread evenly and adhere to the substrate without "de-wetting" to maintain uniform and persistent coverage of the synthesis surface.

Ideally, the boiling point of the film may be greater than about 250 °C, greater than about 275 °C or greater than about 300 °C. The vapor pressure of the film may be less than about 0.02 mmHg, less than about 0.01 mmHg or less than about 0.005 mmHg. Low vapor pressure and high boiling point may minimize film evaporation and may provide long-term persistence under ambient temperature and pressures ranging from ambient (14.7 psi) to moderately low pressures (14.0 psi) used for vacuum-assisted contact lithography.

The log P of the film may be between about -1 and about 3 or between about - 0.5 and about 1.5, which may provide for efficient removal of the released photolysis products from the substrate surface and may also promote adhesion of the film to nonpolar substrates typically used in photolithographic array preparation. The surface tension of the film may be less than 50 dynes/cm² or less than 40 dynes/cm² which may allow uniform film spreading and persistent film coverage of the substrate surface. The viscosity of the film may be less than 150 cPs, less than about 100 cPs or less than about 50 cPs, which may low enough to permit efficient diffusion of released photolysis products and may be high enough to maintain uniform and persistent coverage of the substrate surface,

The film may include but is not limited to, amides (DMF, DMA, NMP), alkyl sulfoxides, sulfolane, trialkylphosphate esters, alkyl phthalate esters, alkyl adipate esters, alkyl mellitate esters, cyclic carbonates, polyethylene glycols, including monoalkyl ethers of polyethylene glycols, dialkylethers of polyethylene glycols, monoalkanoic esters of polyethylene glycols, dialkanoic esters of polyethylene glycols, where the molecular weight is between about 250 daltons and between about 1000 daltons, polyethoxylated polyols, alkyl nitriles, glutaronitrile, adiponitrile, phenylacetonitrile, alkyl cyanoethyl ethers, bis-(2)-cyanoethyl ether, ethylene glycol bis-(2)-cyanoethyl ether, alkyl cyanoacetyl esters, ionic liquids, 3-alkyl-1-methylimidiazolium hexafluorophosphate or mixtures thereof.

More particularly, the film may include, but is not limited to, acetonitrile, glutaronitrile, 2-methylglutaronitrile, adiponitrile, 1,5-dicyanopentane, 1,6-dicyanohexane, 1,4-dicyanobutane, ethyl 2,3-dicyanopropionate, triethyleneglycol diethyl ether, diethylene glycol dibutyl ether, tetraethylene glycol dimethyl ether, tetraethylene glycol diethyl ether, diethylene glycol monohexyl ether, triethyleneglycol monobutyl ether, triethyleneglycol monooctyl ether, tetraethyleneglycol monodecyl, 1,3-dioxan-2-one ether, 1,2-propylene glycol, trimethylene carbonate, propylene carbonate, (4-methyl-1,3-dioxolan-2-one, 4-ethyl-1,3-dioxolan-2-one, butylene carbonate, 4-vinyl-1,3-dioxolan-2-one, 4-isopropyl-1,3-dioxolan-2-one, 4-isobutyl-1,3-dioxolan-2-one, 4-tertbutyl-1,3-dioxolan-2-one, 4-methoxymethyl-1,3-dioxolane, 4-CH₂O-isopropyl-1,3-dioxolan-2-one, 4-CH₂O-isobutyl-1,3-dioxolan-2-one, 4-CH₂O-tertbutyl-1,3-dioxolan-2-one, 4-trimethylsiloxymethyl-1,3-dioxolane, 4-dimethylethylsiloxymethyl-1,3-dioxolane, 4-(t-butyldimethylsiloxy)methyl-1,3-dioxolane, 4-hydroxymethyl-1,3-dioxolane (glycerol carbonate), 4-(2-trimethylsilyloxyethyl)-1,3-dioxolane, 4-(n-butoxymethyl)-1,3-dioxolan-2-one, 4-(2-ethylhexyloxymethyl)-[1,3]dioxolan-2-one, bis(2-cyanoethy) ether, 1,2-bis(2-cyanoethoxy)ethane1,2,3-tris(2-cyanoethoxy)propane, 2-ethylhexanol, 2-(2-ethylhexyloxy)ethanol, 1-[(2-ethylhexyl)oxy]-2-propanol, 3-(2-ethylhexyloxy)-1,2-propanediol, 3-[2-(2-cyanoethoxy)-3-(2-ethylhexyloxy)propoxyl-propionitrile, 3-(2-ethylhexyloxy)-1,2-bis(2-cyanoethoxy)propane, 4-(2-ethylhexyloxy)-3-hydroxybutyronitrile, 3-(2-cyanoethoxy)-4-(2-ethylhexyloxy)butyronitrile, 2-oxo-[1,3]dioxolan-4-ylmethyl 2-ethylhexanoyl ester, 2-(2-ethylhexyloxy)ethanol, 3-(2-ethylhexyloxy)-propionitrile , 2-(2-(2-ethylhexyloxy)ethoxy)ethanol, 3-(2-(2-ethylhexyloxy)ethoxypropanenitrile, 2-cyanoethyl n-hexanoate, 2-cyanoethyl iso-hexanoate, 2-cyanoethyl cyclopentaecarboxylate, 2-cyanoethyl, 2-ethylhexanoate, 2-cyanoethoxyethyl 2-ethylhexyl carbonate, 2-(2-(2-(2-ethylhexyloxy)ethoxy)ethoxy)ethanol, 3-(2-(2-(2-ethylhexyloxy)ethoxy)ethoxy)propanenitrile, 14-ethyl-3,6,9,12-tetraoxaoctadecan-1-ol, 1,1 DME, diglyme, 1,4-dioxane, triglyme, 9-Crown-3, tetraglyme, 12-Crown-4, 15-Crown-5, 5,6 PEG-250 (n~5.6)-DME, 6.0 Hexa-EG-DME, 6.0, 18-Crown-6, PEG-500 (n~11)-DME, 21, PEG-1000 (n~21)-DME, surfynol, tetra(2-hydroxyethyl)diaminoethane, triethyl phosphate, dimethyl phthalate, diethyl phthalate, 1-butyl-3-methylimidazolium PF₆, tris(3-hydroxypropyl)phosphine, 2-octyl-1-dodecanol, 3-mercaptopropane-1,2-diol, 2-(boc-amino)ethanethiol, 4-[(acetyloxy)methyl]-1,3-dioxolan-2-one, 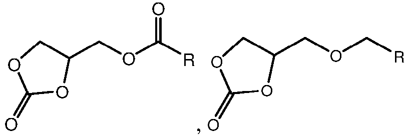 where R = Me, NCCH₂, Me, Et, n-Pr, i-Pr, n-Bu, i-Bu, t-Bu, n-hexyl, n-octyl and phenyl and mixtures thereof.

The film may be a mixture of a monoalkyl ether of a polyethylene glycol, with a molecular weight between about 250 daltons and about 1000 daltons and a cyclic carbonate. The film may include one or more co-reactants, catalysts and promoters. The co-reactants, catalysts and promoters may be a base, acid, reductant, oxidant, sensitizer, photoacid generator, or a polar nucleophilic solvent.

Also disclosed herein are methods of preparing substrates coated with film. Spin-coating is a method for applying polymer thin films to surfaces, and may also be used to apply thin liquid films to suitable substrates. A dilute solution of a high-boiling liquid solvent or mixture thereof may be dissolved in a more volatile carrier solvent such as, for example, acetone, acetonitrile, 2-propanol, methyl ethyl ketone,1,4-dioxane, methyl isobutyl ketone, propylene glycol methyl ether, propylene glycol methyl ether, n-butyl acetate, cyclopentanone, cyclohexanone, methyl isoamyl ketone, N,N-dimethylformamide, ethyl lactate, dimethyl sulfoxide, N-methylpyrrolidone, butyrolactone, propylene carbonate, and the like.

Disclosed herein is a method which minimizes the transfer of film from the substrate to the mask during contact lithography. Imprinting the substrate with narrow, 100- 200 nm thick offsets in a pattern distributed across the surface prevents the mask from making intimate contact with the substrate, while allowing the film to remain on the substrate in between the offsets.

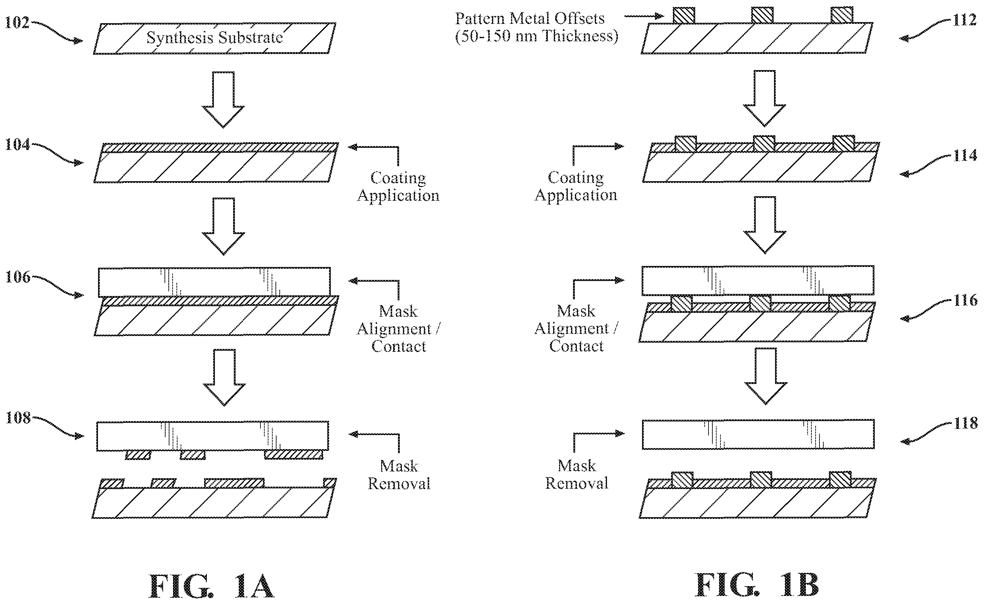

Referring to Fig. 1A, film is applied to substrate **102** which lacks offsets to provide **104.** Mask alignment results in contact with the film at **106,** which results in some film adhering to the mask after the mask is removed at **108.** Contamination of the mask with the film results in lower efficiency of array synthesis.

Referring to Fig. 1B, film is applied to substrate **112** which includes offsets to provide **114.** Note that the length of the offset is greater than the width of the film. Now mask alignment results only in contact with the offset at **116** and thus the mask is not contaminated with any film at **118.** Accordingly, the efficiency of array synthesis is not compromised by mask degradation as the offsets prevent film transfer to the mask.

Further disclosed are methods of synthesizing polymers, methods of synthesizing arrays of polymers and methods of removing photolabile protecting groups. These methods all employ covering or coating the substrate with a thin film of solvent where the thickness is less than 100 µm.

A method of synthesizing a polymer on a substrate is provided. The method includes the steps of providing a substrate comprising functional groups protected by a photolabile protecting group, coating the substrate with a film of solvent wherein the thickness of the film is thinner than about 100 µm, irradiating the photolabile group, removing the film; coupling another monomer having a functional group protected with a photolabile group to the functional group and repeating steps b, c, d and e for one or more repetitions to synthesize the polymer.

A method of synthesizing an array of polymers is provided. The method includes the steps of providing a substrate comprising functional groups protected by a photolabile protecting group, coating the substrate with a film of solvent wherein the thickness of the film is less than about 100 µm, irradiating a selected region of the substrate to remove the photolabile group, removing the film, coupling monomers having a functional group protected with a photolabile group to the functional group and repeating steps b, d and e for one or more repetitions with a different selected region of the substrate to synthesis the array of polymers.

A method of removing a photolabile group from a functional group of a monomer covalently attached to a substrate is provided The method includes the steps of coating the substrate with a film of solvent where the thickness of the film less than about 100 µm and irradiating the photolabile group.

### ARRAY AND POLYMER SYNTHESIS

The methods described, infra, maybe used to prepare polymers and arrays of polymers with the additional steps of covering the substrate with a thin film of solvent of less than 100 nm before removal of the photolabile protecting group and removing the film before adding an additional monomer. The improvements described herein greatly increase the yield and integrity of polymers and arrays of polymer prepared using the methods described below.

Molecules of SAM or in situ synthesized linkers provide sites of attachment for polymer arrays. To distinguish the polymers in arrays, which may be nucleic acids, peptides, polysaccharides, among others, from polymers synthesized in situ as linkers, the polymers in an array are sometimes referred to as array polymers. Attachment can be direct as when an array polymer is linked directly to a tail group of a SAM linker or to a functional group of a functional monomer in a linker synthesized in situ. Attachment can be indirect as when an array polymer is linked to the tail group of SAM linker or to a functional group of a functional monomer in a linker synthesized in situ via an extension linker. If an extension linker is used, array polymers are linked to a functional group of the extension linker. Usually polymers are linked so that the first monomer incorporated into an array polymer is linked to the functional group of an extension linker or to the tail group of a SAM linker or functional group of a monomer of a linker synthesized in situ. The bond formed between an array polymer and a linker can be covalent or non-covalent. Array polymer molecules attach to linker molecules by a single bond joining defined positions of individual polymer and linker molecules such that polymers and linker molecules are uniformly bonded to one another at defined locations on the respective molecules.

Methods and techniques applicable to polymer (including nucleic acid and protein) array synthesis have been described in, WO 00/58516, WO 99/36760 and WO 01/58593, U.S. Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846, 6,428,752, 5,412,087, 6,147,205, 6,262,216, 6,310,189, 5,889,165, and 5,959,098. Nucleic acid probe arrays are described in many of the above patents, but the same techniques are applied to polypeptide arrays and other polymers.

Polymer arrays can be synthesized in a monomer-by-monomer fashion (i.e., polymers are formed by successive coupling of component monomers) or by attachment of preformed polymers. In monomer-by-monomer synthesis the linker (whether an extension linker, SAM linker or in situ synthesized linker) to which the first monomer attaches is initially protected and then deprotected before coupling occurs. The first monomer and successive monomers have at least two functional groups, one to couple to the nascent polymer chain, the other to couple to the next monomer to be added to the chain. The latter function group is typically protected during the coupling step so that polymers are elongated one monomer at a time. The protective group on a monomer is removed after its incorporation to allow coupling to the next monomer.

Monomers can be targeted to specific features of an array by various methods. In one set of methods, arrays are synthesized by a process involving alternating steps of selective activation and coupling. The selective activation removes protecting groups for functional groups either on a linker or on monomers coupled in previous steps generating a pattern of activated regions and inactivated regions on the surface. In the coupling step, a protected monomer is contacted with the support and couples to the functional groups in the activated regions but not at the inactivated regions. By repeating the selective activating and coupling steps different polymers are formed at defined locations on the surface, the sequence and location of the different polymers being defined by the patterns of activated and inactivated regions formed during each activating step and the monomer coupled in each coupling step. Selective deprotection can be achieved with light and photoremovable protective groups or other forms of radiation and corresponding removable protective groups. Selective deprotection can also be achieved using light to remove a photoresist covering a surface of a support from selected regions and subsequently removing protective groups in those regions by chemical treatment, for example use of acid. After removing protective groups from selected regions, the entire surface of a support can be contacted with a protected monomer, which will attach only at the deprotected regions (see, e.g., US20050244755).

Examples of polymer arrays that can be synthesized include nucleic acids, both linear and cyclic, peptides, polysaccharides, phospholipids, heteromacromolecules in which a known drug is covalently bound to any of the above, polyurethanes, polyesters, polycarbonates, polyureas, polyamides, polyethyleneimines, polyarylene sulfides, polysiloxanes, polyimides, and polyacetates. The polymers occupying different features of an array typically differ from one another, although some redundancy in which the same polymer occupies multiple features can be useful as a control. For example, in a nucleic acid array, the nucleic acid molecules within the same feature are typically the same, whereas nucleic acid molecules occupying different features are mostly different from one another.

An exemplary method of synthesis is VLSIPS^{™} (see Fodor et al., Nature 364, 555-556; McGall et al., U.S. Pat. No. 5,143,854; EP 476,014), which entails the use of light or other radiation to direct the synthesis of polymers. Algorithms for design of masks to reduce the number of synthesis cycles are described by Hubbel et al., U.S. Pat. No. 5,571,639 and U.S. Pat. No. 5,593,839.

Performing both peptide and nucleic acid synthesis by photolithographic methods requires closely analogous modifications of conventional solid phase chemical synthesis methods. In each case, the protective group that protects the monomer is changed from a protective group that is suitable for chemical removal to protective group that is photosensitive and can be removed by irradiation. Irradiation is directed e.g., through a mask to a substrate to remove a photosensitive protecting group from known locations on the substrate. The substrate is then exposed to a protected monomer that attaches at the deprotected locations. Then irradiation is again directed through the mask to the substrate exposing known locations (the same or different than before). Then a further protected monomer is supplied, and so forth.

Cho et al., Science 261, 1303-5 (1993) describes the use of a photodeprotection strategy to synthesize an array of oligocarbamates substituted with a variety of side chains. The polymers were synthesized from nitrophenyl carbonate monomers bearing a photosensitive protecting group on a terminal amino moiety. Synthesis is proceeded by photodeprotection of the amino group on an immobilized growing chain allowing coupling of an incoming protected oligocarbamate.

For synthesis of polyureas, a tethered amino group having a radiation-sensitive protecting group is deprotected and treated with a monomer having a first functional group that is an isocyanate and a second functional group that is an amine, protected with a radiation sensitive protecting group. The reaction conditions are adjusted to allow the tethered amine to react with the isocyanate and couple the monomer to the support by forming a urea linkage. The tethered monomer can then be deprotected to liberate or make available the amine functional group that is then free to react with another monomer having an isocyanate and a protected amine. In such a stepwise fashion, a polyurea can be constructed.

Polyamides can be prepared in the same manner as is used for peptide construction. In particular, each monomer has a first carboxylic acid functional group and a second amine, protected with a radiation sensitive protecting group.

The number of different polymers, such as nucleic acids, in an array can be at least 10, 50, 60, 100, 10³, 10⁴, 10⁵, 10⁶, 10⁷, or 10⁸ on a contiguous substrate surface. An array can be subdivided into discrete regions also known as features or cells. Within a cell the polymer molecules are generally of the same type (with the possible exception of a small amount of bleed over from cells and presence of incomplete polymer intermediates of polymer synthesis). It is generally known or determinable, which polymers occupy which regions in an array. The size of individual regions can range from about 1 cm² to 10⁻¹⁰ cm². In some arrays, the individual regions have areas of less than 10⁻¹, 10⁻², 10⁻³, 10¹, 10⁻⁵, 10⁻⁶, 10⁻⁷, 10⁻⁸, 10⁻⁹, or 10⁻¹⁰ cm². The individual regions can be contiguous with one another as can result from VLSIPS methods. The density of regions containing different polymers can thus be greater than 103, 104, 105 or 106 polymers per cm². The polymers can incorporate any number of monomers.

### METHODS OF USING POLYMERS AND ARRAYS

Conventional techniques of organic chemistry, polymer technology, molecular biology (including recombinant nucleic acid techniques), cell biology, biochemistry, and immunology as would be understood by one of the ordinary skill may be used herein. Such conventional techniques include polymer array synthesis, hybridization, ligation, and detection of hybridization using a label. Specific illustrations of suitable techniques can be had by reference to the examples herein below. However, other equivalent conventional procedures can, of course, also be used. Such conventional techniques and descriptions can be found in standard laboratory manuals well known to the skilled artisan.

Many uses for polymers attached to substrates can be contemplated. These uses include gene expression monitoring, profiling, library screening, genotyping and diagnostics. Gene expression monitoring, and profiling methods can be shown in U.S. Pat. Nos. 5,800,992, 6,013,449, 6,020,135, 6,033,860, 6,040,138, 6,177,248 and 6,309,822. Genotyping and uses therefore are shown in U.S. Ser. Nos. 60/319,253, 10/013,598 (U.S. Patent Application Publication 20030036069), and U.S. Pat. Nos. 5,856,092, 6,300,063, 5,858,659, 6,284,460, 6,361,947, 6,368,799 and 6,333,179. Other uses are embodied in U.S. Pat. Nos. 5,871,928, 5,902,723, 6,045,996, 5,541,061, and 6,197,506.

Sample preparation methods are also contemplated. Prior to or concurrent with genotyping, the genomic sample may be amplified by a variety of mechanisms, some of which may employ PCR. See, e.g., U.S. Pat. Nos. 4,683,202, 4,683,195, 4,800,159 4,965,188, and 5,333,675. The sample may be amplified on the array. See, for example, U.S. Pat. No. 6,300,070 and U.S. Ser. No. 09/513,300.

Other suitable amplification methods include the ligase chain reaction (LCR) (e.g., Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and WO 88/10315), self-sustained sequence replication (Guatelli et al., Proc. Nat. Acad. Sci. USA, 87, 1874 (1990) and WO 90/06995), selective amplification of target polynucleotide sequences (U.S. Pat. No. 6,410,276), consensus sequence primed polymerase chain reaction (CP-PCR) (U.S. Pat. No. 4,437,975), arbitrarily primed polymerase chain reaction (AP-PCR) (U.S. Pat. Nos. 5,413,909, 5,861,245) and nucleic acid based sequence amplification (NABSA). (See, U.S. Pat. Nos. 5,409,818, 5,554,517, and 6,063,603,). Other amplification methods that may be used are described in, U.S. Pat. Nos. 5,242,794, 5,494,810, 4,988,617 and in U.S. Ser. No. 09/854,317.

Additional methods of sample preparation and techniques for reducing the complexity of a nucleic sample are described in Dong et al., Genome Research 11, 1418 (2001), in U.S. Pat. Nos. 6,361,947, 6,391,592 and U.S. Ser. Nos. 09/916,135, 09/920,491 (U.S. Patent Application Publication 20030096235), 09/910,292 (U.S. Patent Application Publication 20030082543), and 10/013,598,

Numerous methods for conducting polynucleotide hybridization assays have been well developed. Hybridization assay procedures and conditions will vary depending on the application and are selected in accordance with the general binding methods known to those of skill in the art. Methods and apparatus for carrying out repeated and controlled hybridization reactions have been described in U.S. Pat. Nos. 5,871,928, 5,874,219, 6,045,996 and 6,386,749, 6,391,623.

Detection of hybridization between a ligand and its corresponding receptor by generation of specific signals is also contemplated. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Ser. No. 60/364,731 and in PCT Application PCT/US99/06097 (published as WO99/47964).

Methods and apparatus for signal detection and processing of intensity data are disclosed in, for example, U.S. Pat. Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Ser. No. 60/364,731 and in PCT Application PCT/US99/06097 (published as WO 99/47964).

Conventional biology methods, software and systems may be employed herein. Various computer program products and software for a variety of purposes, such as probe design, management of data, analysis, and instrument operation. See, U.S. Pat. Nos. 5,593,839, 5,795,716, 5,733,729, 5,974,164, 6,066,454, 6,090,555, 6,185,561, 6,188,783, 6,223,127, 6,229,911 and 6,308,170 may be used herein.

Light patterns can also be generated using Digital Micromirrors, Light Crystal on Silicon (LCOS), light valve arrays, laser beam patterns and other devices suitable for direct-write photolithography. See. e.g., U.S. Pat. Nos. 6,271,957 and 6,480,324, incorporated herein by reference.

Methods, devices, and compositions for the formation of arrays of large numbers of different polymer sequences are provided herein. In one aspect, the methods and compositions provided herein involve the conversion of radiation signals into chemical products that are particularly useful in polymer synthesis. The methods and compositions disclosed herein also provide arrays. In one aspect methods, compositions, and devices for the synthesis of an array of different polymers in selected and predefined regions of a substrate are provided. Another aspect includes those arrays and various methods of using them.

Such arrays are used in, for example, in nucleic acid analysis. Polynucleotide or nucleic acid arrays are especially suitable for checking the accuracy of previously elucidated sequences and for detecting mutations and polymorphisms. Polymer arrays are also used in screening studies to evaluate their interaction with, for example, receptors such as antibodies in the case of peptide arrays or with nucleic acids in the case, for example of oligonucleotide arrays. For example, certain embodiments provide for the screening of peptides to determine which if any of a diverse set of peptides has strong binding affinity with a receptor.

In some embodiments, the arrays formed by the present invention are used in competitive assays or other well-known techniques to screen for compounds having certain activities. For example, vast collections of synthetic or natural compounds are immobilized on predefined regions of a substrate. The reaction of the immobilized compounds (or compound) with various test compositions such as the members of a chemical library or a biological extract are tested by dispensing small aliquots of each member of the library or extract to a different region. In one embodiment, a large collection of human receptors is deposited on a substrate, one in each region to form an array. A plant or animal extract is then screened for binding to various receptors of the array.

Nucleic acid sequences can also be immobilized in specific locations or predefined regions of a substrate using the current invention. In some embodiments, such immobilized nucleic acid arrays are used in hybridization assays for gene expression monitoring, nucleic acid amplifications, nucleic acid computation, and nucleic acid analysis in general.

The methods and compositions described herein have certain features in common with the radiation directed methods discussed in U.S. Pat. No. 5,143,854, incorporated herein by reference. The radiation-directed methods discussed in that patent involve activating predefined regions of the substrate and then contacting the substrate with a preselected monomer solution. The predefined regions can be activated with, for example, a light source shown through a mask (much in the manner of photolithographic techniques used in integrated circuit fabrication). Other regions of the substrate remain inactive because they are blocked by the mask from illumination. Thus, a light pattern defines which regions of the substrate react with a given monomer. By repeatedly activating different sets of predefined regions and providing different monomer compositions thereto, a diverse array of polymers is produced on or near the substrate.

According to another aspect, there is no requirement for the use of masks. Predefined regions of the array may be activated by light without the use of photomasks, for example without limitation, by spatial light modulation as discussed in U.S. Pat. No. 6,271,957 and related applications (parent and progeny patents).

According to one aspect, linker molecules having reactive functional groups protected by acid labile protecting groups are provided on the surface of a substrate. In one preferred embodiment of the present invention, a photoacid generator ("PAG") is provided on the surface, preferably in a film with an acid scavenger. This is also called a "resist mixture."

In another aspect, the resist mixture additionally contains a sensitizer. A set of selected regions on the surface of the substrate is exposed to radiation using well-known lithographic methods discussed, for example, in Thompson, L. F.; Willson, C. G.; and Bowden, M. J., Introduction to Microlithography; American Chemical Society, 1994, pp. 212-232.

According to an aspect, acid is generated in the selected regions from the PAG by exposure of the PAG to light of a predetermined wavelength. The generated acid contacts the protected group(s) for long enough and under appropriate conditions to remove the protective group. In accordance with an aspect of the present invention, the protective group is preferably a DMT group and it protects a hydroxyl group. The hydroxyl group can be, for example, part of a substrate, part of a linker, a 5'-hydroxyl group of a nucleotide or deoxynucleotide or a 3'-hydroxyl group of a nucleotide or deoxynucleotide. After sufficient exposure of the protective groups to the acid such that the protective group is removed, but no or substantially no damage is done to any polymer, the surface of the array is stripped, preferably in an appropriate solvent leaving protected and unprotected groups. In one aspect, the protective groups are exposed to the acid for up to 3 hours, such as up to 1 hour, and typically from 2-30 or 5-15 minutes.

Monomers having an acid labile protective group are allowed to react with the exposed groups from the acid treatment. The surface is again coated with one of the resist mixtures described above.

In a particular embodiment, deoxynucleotides having one hydroxyl group with an acid labile protective group and the other with a reactive group, preferably a phosphoramidite group, are allowed to react with the exposed hydroxyl groups from the acid treatment, allowing coupling of the nucleotide to the hydroxyl group. The surface is again coated with one of the resist mixtures described above.

The explanations and illustrations presented herein are intended to acquaint others skilled in the art with the invention, its principles, and its practical application. The specific embodiments of the present disclosure as set forth are not intended to be exhaustive or limit the scope of the disclosure. The scope of the disclosure should be determined with reference to the appended claims, along with the full scope of equivalents to which such claims are entitled. The disclosures of all articles and references, including patent applications and publications, are incorporated by reference for all purposes in their entirety. Other combinations are also possible as will be gleaned from the following claims, which are also hereby incorporated by reference into this written description.

### ILLUSTRATIVE EMBODIMENTS

The following examples are provided to illustrate the disclosed compositions but are not intended to limit the scope thereof. All parts and percentages are by weight unless otherwise stated.

Experiments were performed on silicon substrates (150mm diam./ with 65nm thickness surface layer of silicon dioxide (obtained from Shin-Etsu Microsci). Substrates were cleaned by treatment with Nanostrip (Cyantek-KMG) for 2 hours at room temperature, rinsed thoroughly with deionized water and dried under nitrogen using a Semitool ST-280 Spin Rinser Dryer ("SRD"). The cleaned wafers exhibited surface contact angles of 4(±2) degrees using a VCA2500XE goniometer (AST Products), and were stored under nitrogen, and silanated within 72 hours.

The cleaned substrate surfaces were covalently functionalized with hydroxyalkyl groups by silanation with a 2% (w/v) solution of N-(2-Hydroxyethyl)-N,N- bis[3-(trimethoxysilyl)propyl]amine (Gelest Inc.) in 95:5 ethanol-water for 60 minutes. The substrates were rinsed thoroughly with ethanol, then with deionized water and dried under nitrogen on the SRD. Final surface contact angle of 51(±3) degrees were measured.

### Example 1: General Photolithographic Oligonucleotide Synthesis Procedure Using Thin Films

Photolithographic oligonucleotide synthesis was performed on substrate surfaces using a special "spin-cell" reactor (see: US Patent Application No. 15/200,408), which was interfaced with a commercial oligonucleotide synthesizer (Biolytic Inc.) that was modified to provide programmed reagent delivery to the spin cell.

Using this arrangement, oligonucleotide sequences were synthesized on substrate surfaces by the sequential addition of (DMT or NNBOC)-phosphoramidite building blocks, using standard synthesis protocols which have been adapted for use on flat substrates (G. H. McGall and J. A. Fidanza, Methods in Molecular Biology DNA Arrays Methods and Protocols, edited by J. B. Rampal Humana, Totowa, N.J., 2001, pp. 71-101). All ancillary synthesis reagents used (activator, cap A, cap B, oxidizer and deblock) were standard reagents obtained from Glen Research.

Dimethoxytrityl (DMT)-protected monomers were deblocked using a standard trichloroacetic acid solution after performing coupling, capping and oxidation steps. NNBOC-protected monomers were deblocked by exposure to UV light through an open mask on an NXQ9000 mask aligner (Neutronix-Quintel, Morgan Hill CA). An exposure dose of 1500 mJ/cm² at 365 nm was used. Prior to exposure on the aligner, a thin film of selected coating material (e.g., a high-boiling liquid or mixture of liquids disclosed herein) was applied to the wafer surface as follows: about 4 ml of a solution containing 1-10% of the coating material in a carrier solvent such as propylene glycol methyl ether acetate (PGMEA), was dispensed onto the substrate on the spin cell, covering the surface completely. The substrate was rotated uncovered at 750 rpm for 20 seconds, followed by 1500 rpm for 60 sec. The thickness of the resulting films varied depending on the concentration, viscosity and "adhesive characteristics" of the film composition. Typically, in the case of substances that produced uniform, stable films, a 2-3% solution applied in this way provided a final coating thickness in the range of 20-80 nm (measurements performed using an Alpha-SE Ellipsometer (JA Woolam Co.).

The coated substrate was transferred to the aligner, aligned with the mask, and then brought into hard contact with it. After UV exposure, the mask & wafer were separated, and the wafer was removed from the aligner and returned to the synthesizer spin cell. The synthesis program was resumed, starting with a solvent wash to thoroughly remove the coating material before adding the next monomer in the sequence.

### Example 2: Model Oligonucleotide Synthesis

In order to quantitatively analyze the efficiency of oligonucleotide synthesis, defined length poly-thymidine (T_{N}) oligonucleotides were synthesized, beginning with a fluorescein monomer, on a cleavable linker. In this way, all of the oligonucleotide products could be released from the substrate after synthesis, each having a fluorescent tag attached to the 3'-end, allowing analysis by HPLC.

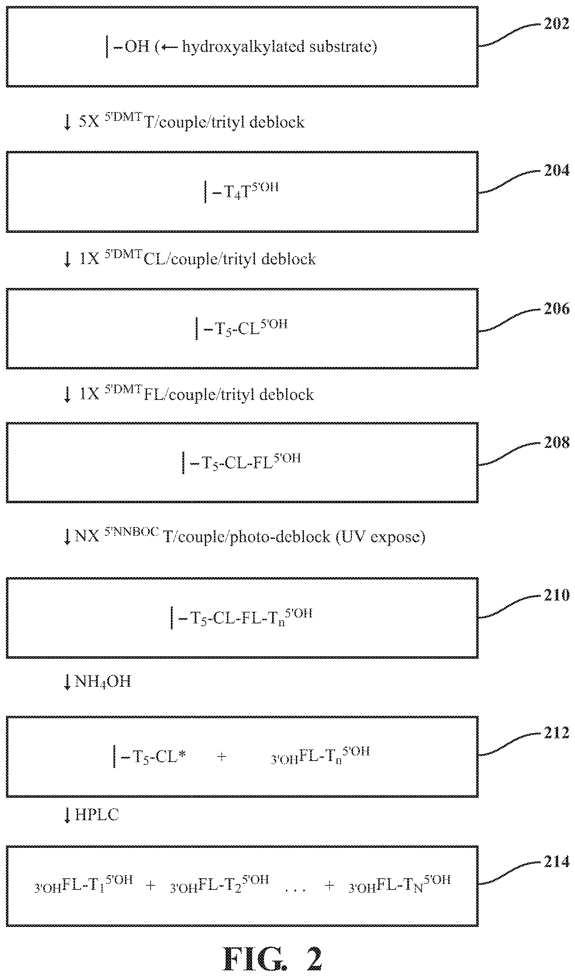

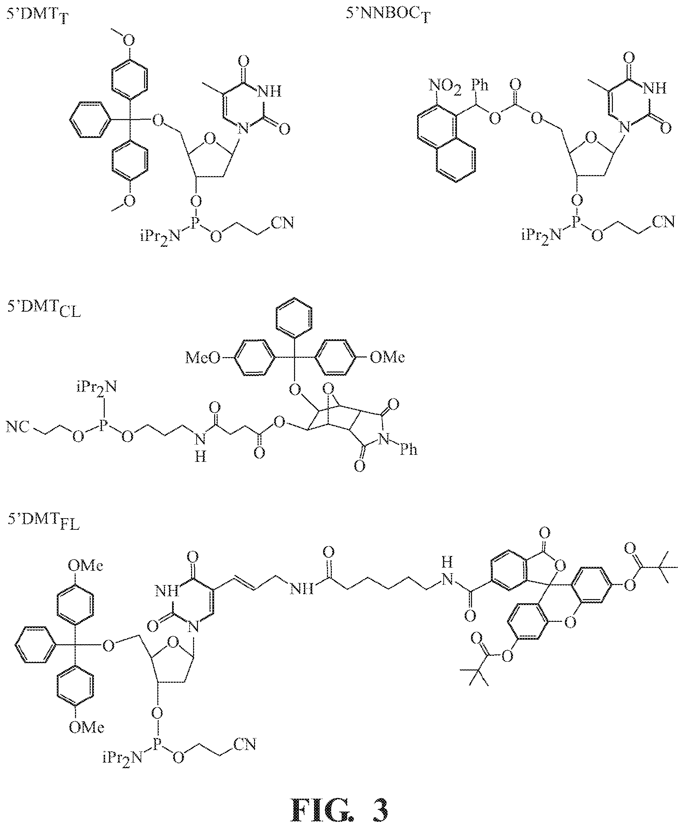

The process is depicted in Fig. 2 and described below with various reagents illustrated in Fig. 3. First, hydroxyalkylated substrate **202** is functionalized by a five repeat synthesis cycles of thymidine-3'-phosphoramidite addition using DMT-thymidine phosphoramidite monomers to add a base layer of T₅ linker sequences to the substrate at **204**. Addition to the base layer **204** of a DMT protected cleavable linker monomer (^{5'DMT}CL) provides T₅-CL^{5'OH} attached to substrate at **206**. The sequence to be analyzed is then begun with the addition of a DMT protected fluorescein linker monomer (^{5'DMT}FL) to provide T₅-CL-FL^{5'OH} attached to substrate at **208**. This is followed by synthesis of the poly-thymidine test sequence, each step employing photo-deblocking, after application of the film on the aligner to yield T₅-CL-FL-Tₙ^{5'OH} attached to substrate at **210.** For comparison, the same test sequence is prepared using "dry" exposures with no film.

### Example 3: Procedure for HPLC Analysis of Model Oligonucleotide Synthesis

After synthesis, the substrate is singulated on a wafer saw (Disco Inc.) into individual 1cm x 1cm chips to be analyzed separately by HPLC. To prepare samples for HPLC analysis, the chip is placed in 1 ml of concentrated NH₄OH solution in a sealed vial and heated at 55°C for 4 hours which cleaves the fluorescein-labelled poly-thymidine oligonucleotide products from the T₅ linker (illustrated in Fig. 2, where T₅-CL-FL-Tₙ^{5'OH} at **210** is hydrolyzed to yield the fluorescein-labelled poly-thymidine oligonucleotide products **212)** and removes protecting groups from the fluorescein and phosphate moieties. The ammonia solution is collected and evaporated to dryness on a Speed-Vac centrifugal evaporator (ThermoFisher). The dried, concentrated products are then re-dissolved in 200 uL of 100mM aqueous sodium phosphate buffer (pH 8).

HPLC analyses were performed on a Shimadzu Prominence HPLC system (Shimadzu Scientific Instruments, Kyoto, Japan) employing an ion-exchange column (DNA-PAC PA-100 (ThermoFisher), and fluorescence detection at 520 nm. Elution was performed with a linear gradient of 0.4 M NaClO₄ in 20 mM Tris pH 8 (or similar buffer system), at a flow rate of 1 mL min⁻¹ to separate the oligonucleotide products. Integration of HPLC peak areas was used to calculate the relative yield of the FL-Tₙ products as shown in Fig. 2 at **214.**

### Example 4: Oligo-T₃ Synthesis Using Bulk Solvent

T₃ was prepared using the procedure described in Examples 1 and 2 except that bulk solvent instead of a film of solvent was used. HPLC analysis was performed as described in Example 3.

**Table 1**

| Summary Effects of Photolysis Solvent on Synthesis Efficiency | | |
|---|---|---|
| Solvent | | Area(TTT)/(Area(TTT)+Area(TT)) |
| 1. None (Dry) | | 14.0 |
| 2. Acetonitrile | | 98.0 |
| 3. Dimethylsulfoxide | | 94.5 |
| 4. Toluene | | 98.0 |
| 5. Hexanes | | 18.0 |
| 6. Hexafluoroisopropanol | | 96.5 |
| 7. 2,6-Lutidine | | 95.5 |
| 8. Methanol | | 94.5 |
| 9. Dioxane | | 92.0 |
| 10. Methyl Isobutyl Ketone | | 95.0 |
| 11. Water | | 22.0 |
| 12. Dichloromethane | | 97.0 |

In most organic solvents, the yield of full-length oligonucleotide product is high (>95%). The results suggest that the ability of the solvent to promote the diffusion of the photolysis product away from the substrate surface during the exposure step, improves the photolytic efficiency. Under dry conditions, and in solvents in which the nitrosoketone photolysis product is insoluble (water, hexane), the efficiency of blocking group photo-removal is significantly lower, thus reducing the yield of full-length oligonucleotide product. Note that any oligomers which are not fully deprotected in any given exposure step can still undergo photolysis in subsequent cycles. But as the synthesis proceeds, the result will be an accumulation of shortened oligonucleotides dominated by the "n-1" - length species.

### Example 5: Comparison of Oligo-T₃ Synthesis Using Bulk Solvent with Oligo-T₃ Synthesis Using Film

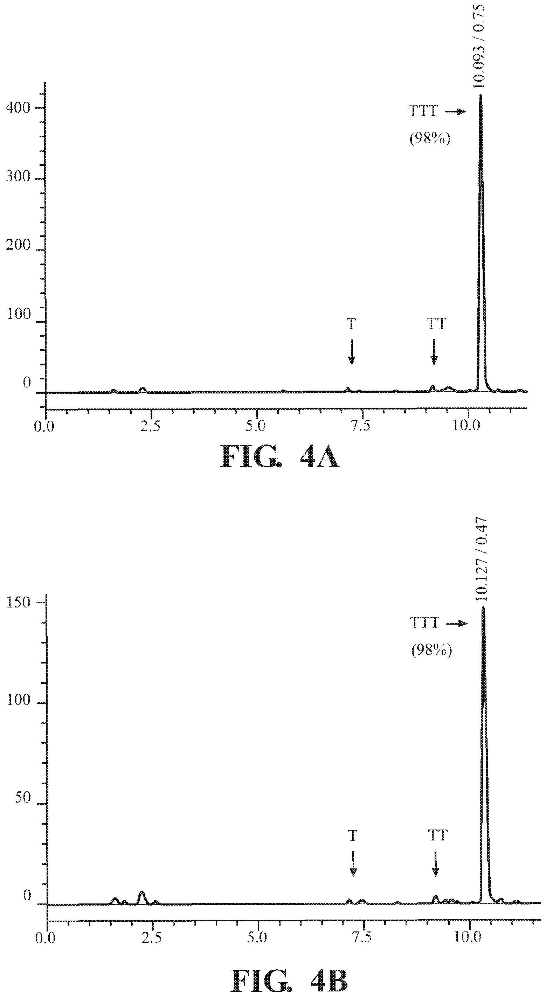

**Oligo-T₃** synthesis in bulk solvent was performed as described in Example 4. **Oligo-T₃** synthesis using a film of 10% propylene carbonate in PGMEA was performed as described in Examples 1 and 2. HPLC analysis was performed as described in Example 3. The HPLC traces illustrated in Fig. 4A and 4B illustrated that oligo-T₃ synthesis using a film of 10% propylene carbonate in PGMEA was as efficient as oligo-T₃ synthesis using bulk solvent.

### Example 6: Synthesis of Ts With No Film

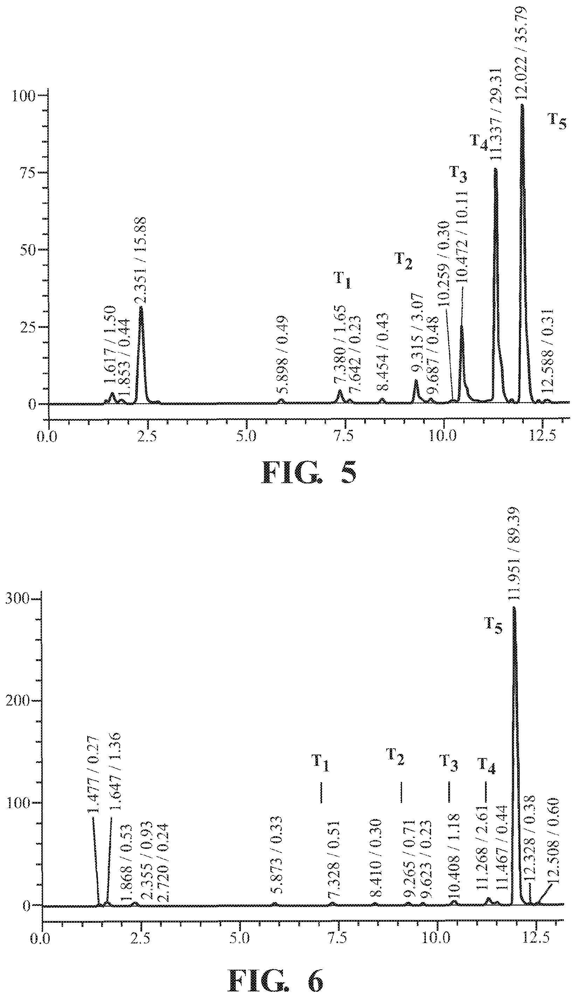

T₅ was prepared using the procedure described in Examples 1 and 2 except that a thin film was not applied. HPLC analysis was performed as described in Example 3. The nominal stepwise efficiency was 87% with N/(N+(N-1) equal to 55%. HPLC analysis of the product is shown in Figure 5.

### Example 7: Synthesis of Ts With Propylene Carbonate and Acetonitrile Film

T₅ was prepared using the procedure described in Examples 1 and 2. HPLC analysis was performed as described in Example 3. A film of 10% propylene carbonate in acetonitrile was applied to the substrate before each photolysis step and the nominal stepwise efficiency was 98% with N/(N+(N-1) equal to 97%. HPLC analysis of the product is shown in Figure 6.

### Example 8: Synthesis of Ts With Diethyleneglycol Ethylhexyl Ether(DEGEH) and Propylene Glycol Methyl Ether Acetate (PGMEA) Film

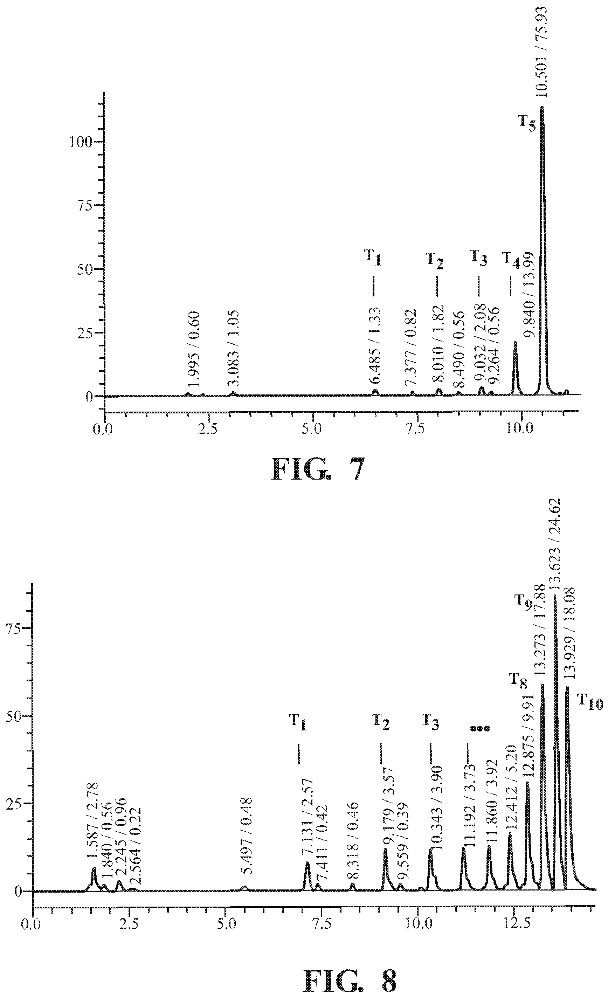

T₅ was prepared using the procedure described in Examples 1 and 2 except that a thin film was not applied. HPLC analysis was performed as described in Example 3. A film of 1% DEGEH in PGMEA was applied to the substrate before each photolysis step and the nominal stepwise efficiency was 95% with N/(N+(N-1) equal to 84%. HPLC analysis of the product is shown in Figure 7.

### Example 9: Synthesis of T₁₀ With No Film

T₁₀ was prepared using the procedure described in Examples 1 and 2 except that a thin film was not applied. HPLC analysis was performed as described in Example 3. The nominal stepwise efficiency was 86% with N/(N+(N-1) equal to 42%. HPLC analysis of the product is shown in Figure 8.

### Example 10: Synthesis of T₁₀ With Diethyleneglycol Ethylhexyl Ether(DEGEH) and Propylene Glycol Methyl Ether Acetate (PGMEA) Film

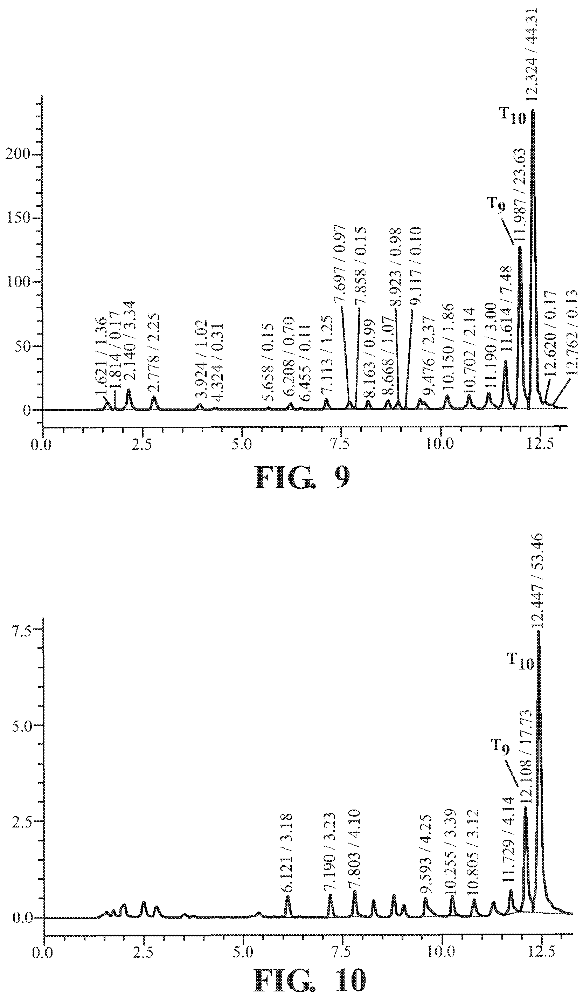

T₁₀ was prepared using the procedure described in Examples 1 and 2. HPLC analysis was performed as described in Example 3. A film of 1% DEGEH and PGMEA was applied to the substrate before each photolysis step and the nominal stepwise efficiency was 93% with N/(N+(N-1) equal to 65%. HPLC analysis of the product is shown in Figure 9.

### Example 11: Synthesis of T₁₀ With 3-(2-(2-Ethylhexyloxy)ethoxypropanenitrile and Propylene Glycol Methyl Ether Acetate (PGMEA) Film

T₁₀ was prepared using the procedure described in Examples 1 and 2. HPLC analysis was performed as described in Example 3. A film of 2% 3-(2-(2-Ethylhexyloxy)ethoxypropanenitrile and PGMEA was applied to the substrate before each photolysis step and the nominal stepwise efficiency was 94% with N/(N+(N-1) equal to 75%. HPLC analysis of the product is shown in Figure 10.

### Example 12: Synthesis of T₁₀ With Surfynol 440 and Propylene Glycol Methyl Ether Acetate (PGMEA) Film

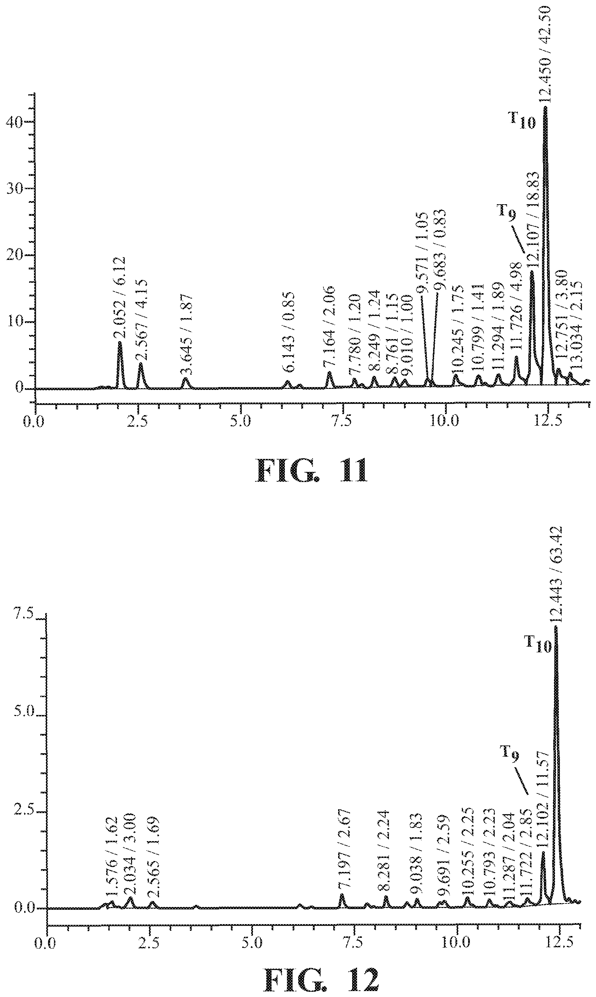

T₁₀ was prepared using the procedure described in Examples 1 and 2. HPLC analysis was performed as described in Example 3. A film of 2% Surfynol 440 and PGMEA was applied to the substrate before each photolysis step and the nominal stepwise efficiency was 94% with N/(N+(N-1) equal to 71%. HPLC analysis of the product is shown in Figure 11.

### Example 13: Synthesis of T₁₀ With Surfynol 440 and 3-Methylglutaronitrile and Propylene Glycol Methyl Ether Acetate (PGMEA) Film

T₁₀ was prepared using the procedure described in Examples 1 and 2. HPLC analysis was performed as described in Example 3. A film of 2% Surfynol 440 and 0.4% 3-methylglutaronitrile in PGMEA was applied to the substrate before each photolysis step and the nominal stepwise efficiency was 96% with N/(N+(N-1) equal to 85%. HPLC analysis of the product is shown in Figure 12.

### Example 14: Synthesis of T₁₀ With Surfynol 440, 4-Methoxymethyl-1,3-dioxolan-2-one (R-(+)) and Propylene Glycol Methyl Ether Acetate (PGMEA) Film

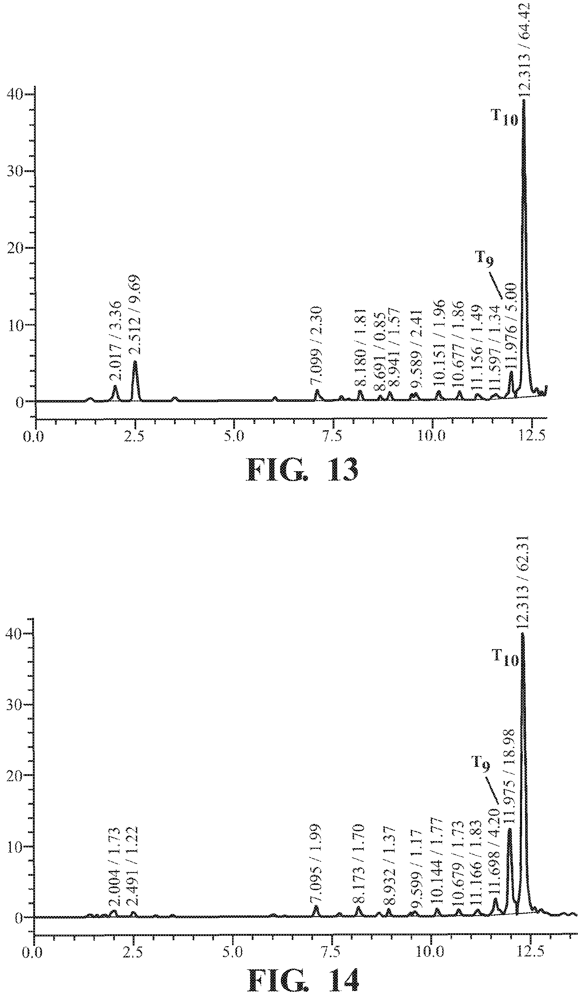

T₁₀ was prepared using the procedure described in Examples 1 and 2. HPLC analysis was performed as described in Example 3. A film of 1.5% Surfynol 440, 0.5% 4-Methoxymethyl-1,3-dioxolan-2-one (R-(+)) and PGMEA was applied to the substrate before each photolysis step and the nominal stepwise efficiency was 96% with N/(N+(N-1) equal to 85%. HPLC analysis of the product is shown in Figure 13.

### Example 15: Synthesis of T₁₀ With Surfynol 440, Diethyl Phthalate and Propylene Glycol Methyl Ether Acetate (PGMEA) Film

T₁₀ was prepared using the procedure described in Examples 1 and 2. HPLC analysis was performed as described in Example 3. A film of 1.5% Surfynol 440, 0.5% diethyl phthalate and PGMEA was applied to the substrate before each photolysis step and the nominal stepwise efficiency was 96% with N/(N+(N-1) equal to 85%. HPLC analysis of the product is shown in Figure 14.

### Example 16: Synthesis of T₁₀ With Surfynol 440, 1.0% Bis(2-cyanoethyl ether) and Propylene Glycol Methyl Ether Acetate (PGMEA) Film

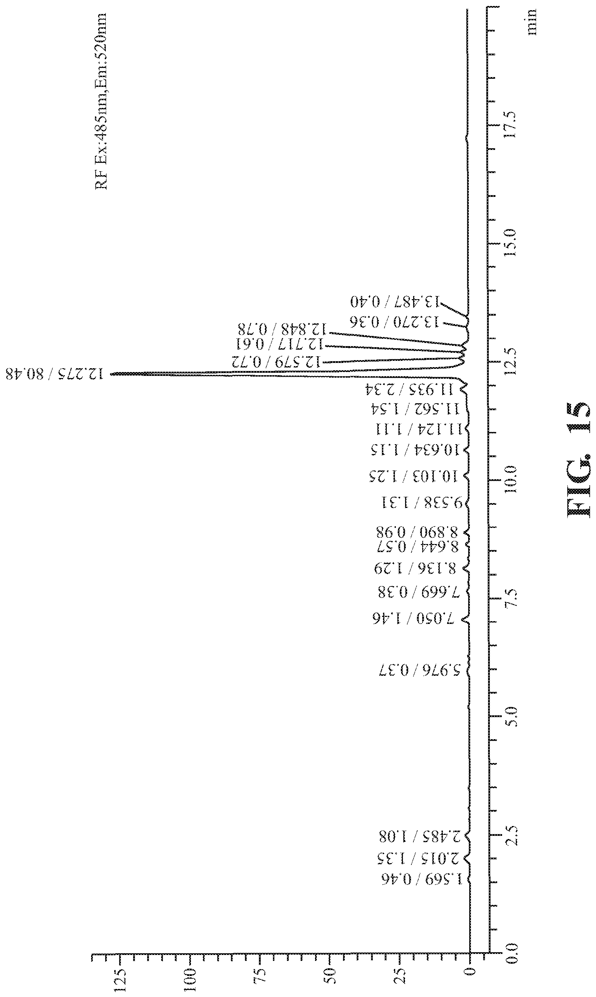

T₁₀ was prepared using the procedure described in Examples 1 and 2. HPLC analysis was performed as described in Example 3. A film of 1.0 % Surfynol 440, 1.0% Bis(2-cyanoethyl ether) and PGMEA was applied to the substrate before each photolysis step and the nominal stepwise efficiency was 96% with N/(N+(N-1) equal to 85%. HPLC analysis of the product is shown in Figure 15.

The present invention also contemplates the following embodiments set out in the below numbered clauses.
1. A substrate comprising a functional group protected with a photolabile group covalently attached to the substrate; and
   a film of solvent thereof covering the substrate, wherein the thickness of the film is less than about 100 µm.
2. The substrate of clause 1, wherein the thickness of the film is less than about 100 nm.
3. The substrate of clause 1, wherein the photolabile group is NNBOC, NNPOC, MENPOC, DMBOC, PYMOC, NPPOC or DEACMOC.
4. The substrate of clause 1, wherein the substrate comprises a flat surface which includes one or more offsets.
5. The substrate of clause 5, wherein the offset comprises a metal, metal oxide or metal nitride.
6. The substrate of clause 6, wherein the offset comprises tantalum, gold, tungsten or chromium.
7. The substrate of clause 1, wherein the film comprises an amide, an alkyl sulfoxide, a sulfolane, an trialkylphosphate ester, an alkyl phthalate ester, alkyl adipate ester, an alkyl mellitate ester, a cyclic carbonate, a polyethylene glycol, including a monoalkyl ether of a polyethylene glycol, a dialkylether of a polyethylene glycol, a monoalkanoic ester of a polyethylene glycol, a dialkanoic ester of a polyethylene glycol, where the molecular weight is between about 250 daltons and between about 1000 daltons, a polyethoxylated polyol, an alkyl nitrile, glutaronitrile, adiponitrile, phenylacetonitrile, an alkyl cyano ethyl ether, bis-(2)-cyanoethyl ether, ethylene glycol bis-(2)-cyanoethyl ether, alkyl cyanoacetyl esters, an ionic liquid, 3-alkyl-1-methylimidiazolium hexafluorophosphate or mixtures thereof.
8. The substrate of clause 1, wherein the film comprises a mixture of a monoalkyl ether of a polyethylene glycol, with a molecular weight between about 250 daltons and about 1000 daltons and a cyclic carbonate.
9. The substrate of clause 1, wherein the boiling point of the film is greater than about 250 °C, greater than about 275 °C or greater than about 300 °C.
10. The substrate of claim 1, wherein the vapor pressure of the film is less than about 0.02 mmHg, less than about 0.01 mmHg or less than about 0.005 mmHg.
11. The substrate of claim 1, wherein the log P of the film is between about -1 and about 2 or between about -0.5 and about 1.5.
12. The substrate of claim 1, wherein the surface tension of the film is less than 50 dynes/cm² or less than 40 dynes/cm²
13. The substrate of claim 1, wherein the viscosity of the film is less than 150 cPs, less than about 100 cPs or less than about 50 cPs.
14. The substrate of claim 1, wherein the film includes one or more co-reactants.
15. The substrate of claim 1, wherein the co-reactant is a base, acid, reductant, oxidant, sensitizer, photoacid generator, or a polar nucleophilic solvent.
16. A method of making a substrate comprising a functional group protected with a photolabile group covalently attached to the substrate coated with a film comprising spin coating the substrate with the film, wherein the thickness of the film is less than about 100 µm.
17. A method of synthesizing a polymer on a substrate comprising:
   (a) providing a substrate comprising functional groups protected by a photolabile protecting group;
   (b) coating the substrate with a film of solvent wherein the thickness of the film is less than about 100 µm;
   (c) irradiating the photolabile group;
   (d) removing the film;
   (e) coupling another monomer having a functional group protected with a photolabile group to the functional group; and
   (f) repeating steps b, c, d and e for one or more repetitions to synthesis the polymer.
18. A method of synthesizing an array of polymers comprising:
   (a) providing a substrate comprising functional groups protected by a photolabile protecting group;
   (b) coating the substrate with a film of solvent wherein the thickness of the film is less than about 100 µm;
   (c) irradiating a selected region of the substrate to remove the photolabile group;
   (d) removing the film;
   (e) coupling monomers having a functional group protected with a photolabile group to the functional group; and
   (f) repeating steps b, d and e for one or more repetitions with a different selected region of the substrate to synthesis the array of polymers.
19. A method of removing a photolabile group from a functional group of a monomer covalently attached to a substrate comprising:
   coating the substrate with a film of solvent wherein the thickness of the film is less than about 100 µm; and
   irradiating the photolabile group.

## Claims

1. A method of removing a photolabile group from a functional group of a nucleotide or nucleoside covalently attached to a substrate comprising:
covering the substrate and the covalently attached nucleotide or nucleoside with solvent wherein the thickness of the solvent is less than about 100nm and the boiling point of the solvent is greater than 250°C; and
irradiating the photolabile group;
wherein
the photolabile group is an ortho nitro benzyl protecting group and the functional group is the 3' and 5' hydroxyl groups of the nucleotides and nucleosides.

2. The method of claim 1, wherein the ortho nitro benzyl protecting group is nitroveratryloxycarbonyl (NVOC), α-methyl-2-nitroveratryloxycarbonyl (MeNVOC), 2-nitropiperonyloxycarbonyl (NPOC), α-methyl-2-nitropiperonyloxycarbonyl (MeNPOC), 2-nitronaphth-1-ylmethyloxycarbonyl (NNPOC), 2-nitronaphth-1-ylbenzyloxycarbonyl (NNBOC), 6,-methoxy, 2-nitronaphth-1-ylbenzyloxycarbonyl (6-methoxy NNBOC), α-methyl-2-nitronaphth-1-ylmethyloxycarbonyl, α-phenyl-2-nitronaphth-1-ylmethyloxycarbonyl, 2,6-dinitrobenzyloxycarbonyl (DNBOC), α-methyl-2,6-dinitrobenzyloxycarbonyll (MeDNBOC), 2-(2-nitrophenyl)ethyloxycarbonyl (NPEOC), 2-methyl-2-(2-nitrophenyl)ethyloxycarbonyl (NPPOC).

3. The method of claim 1, wherein the ortho nitro benzyl protecting group is 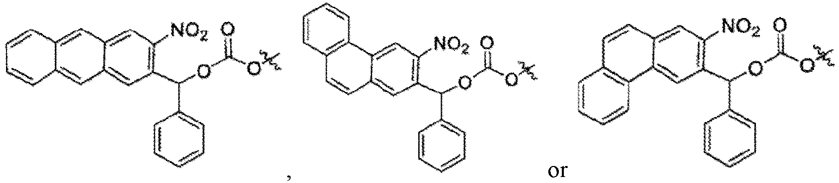 optionally substituted with one or more methoxy groups.

4. The method of claim 1, wherein the substrate further comprises one or more offsets attached to the surface wherein the height of the one or more attached offsets from the substrate surface is between 100 nanometers and 200 nanometers.

5. The method of any preceding claim, wherein the solvent comprises a mixture of monoalkyl ethers of polyethylene glycol and a cyclic carbonate wherein the mixture of monoalkyl ethers of polyethylene glycol have a molecular weight between 250 daltons and 1000 daltons.

6. The method of any preceding claim, wherein the vapor pressure of the solvent is less than 0.02 millimeter of mercury.

7. The method of any preceding claim, wherein the log P of the uniform layer of non-aqueous liquid solvent is between -1 and 2.

8. The method of any preceding claim, wherein the surface tension of the solvent is less than 50 dynes/square centimeter.

9. The method of any preceding claim, wherein the solvent has a viscosity of less than 150 centipoise at 25 °C and 1 atmosphere pressure.

10. The method of claim 1, wherein the solvent comprises 4-methoxymethyl-1,3-dioxolane, 4-isopropoxymethyl-1,3-dioxolan-2-one, 4-isobutoxymethyl-1,3-dioxolan-2-one, **4-**trimethylsiloxymethyl-1,3-dioxolane, 4-dimethylethylsiloxymethyl-1,3-dioxolane, 4-(t-butyldimethylsiloxy)methyl-1,3-dioxolane, 4-hydroxymethyl-1,3-dioxolane (glycerol carbonate), 4-(2-trimethylsilyloxyethyl)-1,3-dioxolane, 4-(n-butoxymethyl)-1,3-dioxolan-2-one, 4-(2-ethylhexyloxymethyl)-[1,3]dioxolan-2-one, bis(2-cyanoethy) ether, 1,2-bis(2-cyanoethoxy)ethane, 1,2,3-tris(2-cyanoethoxy)propane, 2,4,7,9-tetramethyl-5-decyn-4,7-diol, 4-[(acetyloxy)methyl]-1,3-dioxolan-2-one, 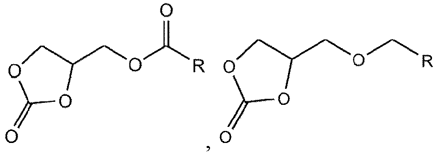 wherein R is -CH₂CN, -CH₃, -CH₂CH₃, -CH₂CH₂CH₃, -CH(CH₃)₂, -CH₂(CH₂)₂CH₃, -CH₂CH(CH₃)₂, - C(CH₃)₃, -CH₂(CH₂)₄CH₃, -CH₂(CH₂)₆CH₃ or -C₆H₅.

11. The method of claim 1, wherein the uniform layer of non-aqueous liquid solvent comprises 4-methoxymethyl-1,3-dioxolane, bis(2-cyanoethy) ether, and 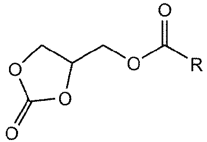 wherein R is -CH(CH₃)₂.

12. The method of claim 1, wherein the uniform layer of non-aqueous liquid solvent comprises 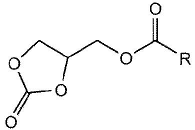 wherein R is -CH(CH₃)₂.

13. The method of claim 1 further comprising transferring the surface to an aligner; and aligning the surface with a mask prior to irradiating the surface.

14. The method of claim 1, wherein irradiating the surface comprise an exposure dose of 1500 mJ/cm₂ at 365 nm.
